# EUROPÄISCHE PATENTSCHRIFT

(11) **EP 0 539 806 B1**
(45) Veröffentlichungstag und Bekanntmachung des Hinweises auf die Patenterteilung: **04.01.1995**
(21) Anmeldenummer: 92117675.6
(22) Anmeldetag: 16.10.1992
(51) Int. Cl.: C07D 285/16, A61K 31/54

(54) **Thiadiazinone und sie enthaltende Arzneimittel, insbesondere mit Positiv-Inotroper und vasodilatierender Wirksamkeit**
Thiadiazinones and medicines containing them, having in particular a positive inotropic and vasodilating activity
Thiadiazinones et médicaments les contenant, ayant en particulier une activité inotrope positive et vasodilatatrice

(30) Priorität: 23.10.1991 DE 4134893
(43) Veröffentlichungstag der Anmeldung: 05.05.1993
(73) Patentinhaber: MERCK PATENT GmbH, D-64271 Darmstadt (DE)
(72) Erfinder: Jonas, Rochus, Dr., D-6100 Darmstadt (DE); Jonas, Rochus, Dr., D-6100 Darmstadt (DE); Leibrock, Joachim, Dr., D-6102 Pfungstadt (DE); Schliep, Hans-Jochen, Dr., D-6109 Mühltal-Traisa (DE); Seyfried, Christoph, Dr., D-6104 Seeheim-Jugenheim (DE); Wolf, Michael, Dr., D-6100 Darmstadt (DE)

(56) Entgegenhaltungen:
- EP-A- 0 080 296

## Beschreibung

Die Erfindung betrifft neue Thiadiazinonderivate der Formel I 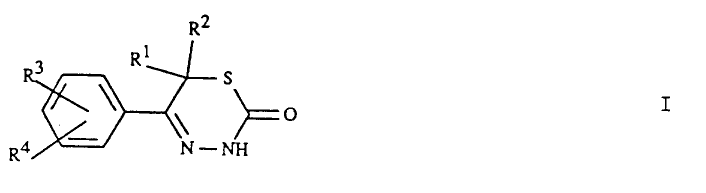
worin
- R¹ und R²: jeweils unabhängig voneinander H oder A,
- R³: H, OA oder O-CₘH₂ₘ₊₁₋ₙXₙ,
- R⁴: -O-CₘH₂ₘ₊₁₋ₙXₙ,
- X: F oder Cl,
- A: Alkyl mit 1-6 C-Atomen,
- m: 1, 2, 3, 4, 5 oder 6 und
- n: 1, 2, 3, 4, 5, 6, 7, 8, 9, 10, 11, 12 oder 13
bedeuten,
sowie deren Salze.

Strukturell ähnliche Thiadiazinone sind aus EP-A-0 080 296 bekannt. Die Verbindungen des Standes der Technik besitzen ebenso wie die hier beanspruchten Verbindungen, einen Phenylrest in 5-Position, welcher ein bis vier weitere Substituenten aufweisen kann, die beispielsweise F, Cl, Br, CF₃ oder Methoxy sein können. Die Position 6 der bereits bekannten Thiadiazinone ist vorwiegend unsubstituiert. Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I bei guter Verträglichkeit wertvolle pharmakologische Eigenschaften besitzen. Insbesondere zeigen sie eine Wirkung auf die Herzkraft (positiv inotrope Wirksamkeit); ferner wirken die Substanzen vasodilatierend und fördern daher die Durchblutung. Die vasodilatierende und die Herzwirkung kann z.B. an narkotisierten oder wachen Hunden, Katzen, Affen oder Minischweinen, die positiv inotrope Wirkung auch an isolierten Herzpräparationen (z.B. Vorhof, Papillarmuskel oder perfundiertes Ganzherz) von Ratte, Meerschweinchen, Katze oder Hund ermittelt werden, z.B. nach Methoden, wie sie in Arzneimittelforschung, Band 31 (I) Nr. 1a (1981), Seiten 141 bis 170, oder von Schliep et al. im 9th International Congress of Pharmacol., London, Abstracts of papers 9P, beschrieben sind.

Weiter können die Verbindungen zur Behandlung von asthmatischen Erkrankungen eingesetzt werden. Die antiasthmatische Wirkung kann z.B. nach der Methode von T. Olsson, Acta Allergologica 26, 438-447 (1971), bestimmt werden.

Die Verbindungen wirken ferner cerebroprotektiv, können zur Behandlung von Gedächtnisstörungen eingesetzt werden und haben antidepressive sowie antiphlogistische Eigenschaften.

Die Verbindungen können daher als Arzneimittelwirkstoffe in der Human- und Veterinärmedizin verwendet werden. Ferner können sie als Zwischenprodukte zur Herstellung weiterer Arzneimittelwirkstoffe verwendet werden.

Gegenstand der Erfindung sind dementsprechend die Verbindungen der Formel I sowie ein Verfahren zu ihrer Herstellung, dadurch gekennzeichnet, daß man eine Verbindung der Formel II, 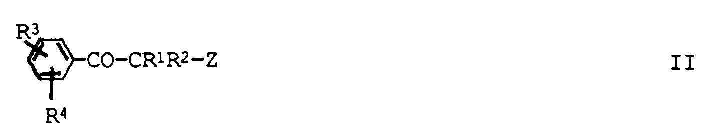
worin R¹, R², R³ und R⁴ die angegebenen Bedeutungen haben und
- Z: Br, Cl, I oder eine reaktionsfähig veresterte OH-Gruppe bedeutet,
mit einer Verbindung der Formel III

H₂N-NH-CS-OR⁵ III

worin
- R⁵: A, Ammonium, Na oder K bedeutet
und A die angegebene Bedeutung hat,
umsetzt
und/oder daß man gegebenenfalls eine Verbindung, die der Formel I entspricht, jedoch anstelle von R³ und/oder R⁴ eine oder zwei freie OH-Gruppen enthält, mit einer Verbindung der Formel R³-Z bzw. R⁴-Z, worin R³, R⁴ und Z die angegebenen Bedeutungen haben, umsetzt und/oder eine Base der Formel I durch Behandeln mit einer Säure in eines ihrer Salze umwandelt.

Vor- und nachstehend haben die Reste R¹ bis R⁴, R⁵, A, X und Z sowie die Parameter m und n die bei den Formeln I, II und III angegebenen Bedeutungen, wenn nicht ausdrücklich etwas anderes angegeben ist.

In den Formeln ist Alkyl vorzugsweise unverzweigt, hat vorzugsweise 1, 2, 3 oder 4 C-Atome und steht bevorzugt für Methyl, ferner bevorzugt für Ethyl oder Propyl, weiterhin bevorzugt für Isopropyl, Butyl, Isobutyl, sek.-Butyl, tert.-Butyl, aber auch für n-Pentyl oder Isopentyl.

Alkoxy ist vorzugsweise unverzweigt, hat vorzugsweise 1, 2 oder 3 C-Atome und steht bevorzugt für Methoxy, ferner bevorzugt für Ethoxy oder Propoxy, weiterhin z.B. für Isopropoxy, Butoxy, Isobutoxy, sek.-Butoxy, tert.-Butoxy, Pentoxy oder Isopentoxy.

Die Reste R¹, R², R³ und R⁴ haben im einzelnen die folgenden bevorzugten Bedeutungen: R¹ H; R² Methyl oder Ethyl; R³ Methoxy und R⁴ Difluormethoxy. Falls die Reste R³ und/oder R⁴ von H verschieden sind, stehen sie vorzugsweise in 3- oder 4-Stellung des Phenylrings. Der Rest X ist vorzugsweise F.

Die Parameter m und n sind bevorzugt 1, 2 oder 3, wobei n ferner noch vorzugsweise 4, 5, 6 oder 7 sein kann.

Gegenstand der Erfindung sind insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ia bis Id ausgedruckt werden, die der Formel I entsprechen und worin die nicht näher bezeichneten Reste die bei der Formel I angegebene Bedeutung haben, worin jedoch
in Ia R³ in 4- und R⁴ in 3-Stellung des Phenylrings steht,
- R¹: H,
- R²: H oder Alkyl,
- R³: OCHF₂ und
- R⁴: OA bedeuten;
in Ib R³ in 4- und R⁴ in 3-Stellung des Phenylrings steht,
- R¹: H,
- R²: Methyl oder Ethyl,
- R³: OCHF₂ und
- R⁴: OA bedeuten;
in Ic R³ in 4- und R⁴ in 3-Stellung des Phenylrings steht,
- R¹: H,
- R²: Methyl oder Ethyl,
- R³: OCHF₂, OCF₃, OC₂H₅₋ₙFₙ, wobei n = 1, 2, 3, 4 oder 5 ist und
- R⁴: OA bedeuten;
in Id R³ in 4- und R⁴ in 3-Stellung des Phenylrings steht
- R¹: H,
- R²: H, Methyl oder Ethyl,
- R³: OCHF₂, OCF₃, OC₂H₅₋ₙFₙ, OC₃H₇₋ₙFₙ, OC₄H₉₋ₙFₙ, wobei n = 1, 2, 3, 4 oder 5, für OC₃H₇₋ₙFₙ und OC₄H₉₋ₙFₙ auch 6 oder 7 und für OC₄H₉₋ₙFₙ ebenfalls 8 oder 9 ist und
- R⁴: OMe oder OEt bedeuten.

Die Verbindungen der Formel I werden im übrigen nach an sich bekannten Methoden hergestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

In den Verbindungen der Formel II haben R¹, R², R³ und R⁴ die angegebenen Bedeutungen, während Z bevorzugt für Cl oder Br steht. Falls X eine reaktionsfähig veresterte OH-Gruppe bedeutet, so ist diese vorzugsweise Alkylsulfonyloxy mit 1-6 C-Atomen, z.B. Methansulfonyloxy oder Arylsulfonyloxy mit 6-10 C-Atomen, z.B. Benzol-, p-Toluol- oder 1- oder 2-Naphthalinsulfonyloxy.

In den Verbindungen der Formel III steht R⁵ bevorzugt für Methyl oder Ethyl, aber auch für Na, K oder Ammonium.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt. Andererseits ist es möglich, die Reaktion stufenweise durchzuführen, wobei man weitere Zwischenprodukte isolieren kann.

Die Ausgangsstoffe der Formeln II und III sind teilweise bekannt. Sofern sie nicht bekannt sind können sie nach an sich bekannten Methoden hergestellt werden. Die Ketone der Formel II sind beispielsweise durch Friedel-Crafts-Synthese aus den entsprechenden Phenylderivaten mit Verbindungen der Formel Y-CO-CR¹R²-Z, wobei Y Cl oder Br bedeutet, zugänglich.

Im einzelnen erfolgt die Umsetzung der Ketone der Formel II mit den Verbindungen der Formel III in Gegenwart oder Abwesenheit eines inerten Lösungsmitteln bei Temperaturen zwischen etwa -20 und etwa +150°, vorzugsweise zwischen 20 und 100°. Als Lösungsmittel eignen sich beispielsweise Kohlenwasserstoffe wie Benzol, Toluol, Xylole oder Mesitylen; halogenierte Kohlenwasserstoffe wie Dichlormethan, Trichlorethylen oder Chlorbenzol; Alkohole wie Methanol, Ethanol oder Isopropanol; Glykole und Glykolether wie Ethylenglykol, Diethylenglykol, 2-Methoxyethanol; Nitrile wie Acetonitril; Ether wie Tetrahydrofuran oder Dioxan; Amide wie Dimethylformamid (DMF); Sulfoxide wie Dimethylsulfoxid. Auch Gemische dieser Lösungsmittel sind geeignet.

Ebenso ist es möglich eine Verbindung, die der Formel I entspricht, jedoch anstelle von R³ und/oder R⁴ eine oder zwei freie OH-Gruppen enthält, mit einer Verbindung der Formel R³-Z bzw. R⁴-Z, worin R³, R⁴ sowie Z die angegebenen Bedeutungen haben, umzusetzen. Die Veretherung der OH-Gruppen erfolgt nach an sich bekannten Methoden, wie sie in Standardwerken der chemischen Literatur (z.B. in Houben-Weyl, Methoden der Organischen Chemie, Georg Thieme Verlag, Stuttgart oder in Organic Reactions, John Wiley & Sons Inc., New York) beschrieben sind, und zwar unter Reaktionsbedingungen, wie sie für die genannten Umsetzungen bekannt und geeignet sind. Dabei kann auch von an sich bekannten, hier nicht näher erwähnten Varianten Gebrauch gemacht werden.

Verbindungen der Formel I können eine oder mehrere Asymmetriezentren enthalten. In diesem Fall liegen sie gewöhnlich in racemischer Form vor. Erhaltene Racemate können nach an sich bekannten Methoden mechanisch oder chemisch in ihre optischen Antipoden getrennt werden. Vorzugsweise werden aus dem racemischen Gemisch durch Umsetzung mit einem optischaktiven Trennmittel Diastereomere gebildet.

Natürlich ist es auch möglich, optisch aktive Verbindungen der Formel I nach den oben beschriebenen Methoden zu erhalten, indem man Ausgangsstoffe verwendet, die bereits optisch aktiv sind.

Gegenstand der Erfindung ist ferner die Verwendung der Verbindungen der Formel I und ihrer physiologisch unbedenklichen Salze zur Herstellung pharmazeutischer Zubereitungen, insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignet Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Dragees, Kapseln, Sirupe, Säfte oder Tropfen, zur rektalen Anwendung Suppositorien, zur parenteralen Anwendung Lösungen, vorzugsweise ölige oder wässerige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstofe enthalten, z.B. ein oder mehrere Vitamine.

Die Verbindungen der Formel I können bei der Bekämpfung von Krankheiten, insbesondere von Herzinsuffizienz, sowie bei der therapeutischen Behandlung des menschlichen oder tierischen Körpers verwendet werden.

Dabei werden die erfindungsgemäßen Substanzen in der Regel in Analogie zu bekannten positiv inotrop wirksamen Substanzen wie Amrinon verabreicht, vorzugsweise in Dosierungen zwischen etwa 1 und 100 mg, insbesondere zwischen 2 und 20 mg pro Dosierungseinheit. Die tägliche Dosierung liegt vorzugsweise zwischen etwa 0,02 und 2 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt. Im Vergleich zu den bisher zur Therapie der Herzinsuffizienz verwendeten Digitalis-Glykosiden zeichnen sich die Verbindungen der Formel I durch verbesserte therapeutische Breite und periphere Entlastung aus.

In den nachfolgenden Beispielen bedeutet "übliche Aufarbeitung":
Man gibt, falls erforderlich, Wasser oder verdünnte Natronlauge hinzu, extrahiert mit einem organischen Lösungsmittel wie Ethylacetat, Chloroform oder Dichlormethan, trennt ab, trocknet die organische Phase über Natriumsulfat, filtriert, dampft ein und reinigt durch Chromatograhie und/oder Kristallisation.

Vor- und nachstehend sind alle Temperaturen in Celsiusgraden angegeben.

### Beispiel 1

Eine Lösung von 7,0 g 1-(3-Methoxy-4-difluormethoxy-phenyl)-2-brombutan-1-on [erhältlich durch Veretherung von 1-(4-Hydroxy-3-methoxyphenyl)-butan-1-on mit Chlordifluormethan und nachfolgende Bromierung] in 50 ml Acetonitril wird mit 2,6 g Hydrazinthioameisensäuremethylester zwei Std. gekocht.

Anschließend wird das Lösungsmittel im Vakuum entfernt und wie üblich aufgearbeitet. Man erhält 5-(3-Methoxy-4-difluormethoxyphenyl)-6-ethyl-3,6-dihydro-1,3,4-thiadiazin-2-on, F. 97°.

Analog erhält man durch Umsetzung von Hydrazinthioameisensäuremethylester mit
1-(3-Methoxy-4-trifluormethoxy-phenyl)-2-brombutan-1-on:
5-(3-Methoxy-4-trifluormethoxyphenyl)-6-ethyl-3,6-dihydro-1,3,4-thiadiazin-2-on;
1-(3-Methoxy-4-trifluormethoxy-phenyl)-2-brompropan-1-on:
5-(3-Methoxy-4-trifluormethoxyphenyl)-6-methyl-3,6-dihydro-1,3,4-thiadiazin-2-on;
1-(3-Methoxy-4- difluormethoxy-phenyl)-2-brompropan-1-on:
5-(3-Methoxy-4-difluormethoxyphenyl)-6-methyl-3,6-dihydro-1,3,4-thiadiazin-2-on;
1-[3-Methoxy-4-(1,1,2,2-tetrafluorethoxy)-phenyl]-2-brombutan-1-on:
5-[3-Methoxy-4-(1,1,2,2-tetrafluorethoxy)-phenyl]-6-ethyl-3,6-dihydro-1,3,4-thiadiazin-2-on;
1-(3-Methoxy-4-chlormethoxy-phenyl)-2-brombutan-1-on:
5-(3-Methoxy-4-chlormethoxy-phenyl)-6-ethyl-3,6-dihydro-1,3,4-thiadiazin-2-on;
1-(3-Methoxy-4-(chlormethoxy-phenyl)-2-brompropan-1-on:
5-(3-Methoxy-4-chlormethoxy-phenyl)-6-methyl-3,6-dihydro-1,3,4-thiadiazin-2-on;
1-(3-Methoxy-4-pentachlorethoxy-phenyl)-2-brombutan-1-on:
5-(3-Methoxy-4-pentachlorethoxy-phenyl)-6-ethyl-3,6-dihydro-1,3,4-thiadiazin-2-on;
1-(3-Methoxy-4-trifluormethoxy-phenyl)-2-brompentan-1-on:
5-(3-Methoxy-4-trifluormethoxy-phenyl)-6-propyl-3,6-dihydro-1,3,4-thiadiazin-2-on;
1-(3-Methoxy-4-difluormethoxy-phenyl)-2-brompentan-1-on:
5-(3-Methoxy-4-difluormethoxy-phenyl)-6-propyl-3,6-dihydro-1,3,4-thiadiazin-2-on;
1-[3-Methoxy-4-(1,1,2-trifluorethoxy)-phenyl]-2-brombutan-1-on:
5-[3-Methoxy-4-(1,1,2-trifluorethoxy)-phenyl]-6-ethyl-3,6-dihydro-1,3,4-thiadiazin-2-on;
1-[3-Methoxy-4-(1,1,2-trifluorethoxy)-phenyl]-2-brompropan-1-on:
5-[3-Methoxy-4-(1,1,2-trifluorethoxy)-phenyl]-6-methyl-3,6-dihydro-1,3,4-thiadiazin-2-on.

### Beispiel 2

Analog Beispiel 1 erhält man durch Umsetzung von Hydrazinthioameisensäuremethylester mit 1-(4-Difluormethoxy-3-methoxyphenyl)-2-bromethan-1-on: 5-(3-Methoxy-4-difluormethoxyphenyl)-3,6-dihydro-1,3,4-thiadiazin-2-on, F. 120°.

Analog erhält man durch Umsetzung von Hydrazinthioameisensäuremethylester mit
1-(3-Methoxy-4-trifluormethoxy-phenyl)-2-bromethan-1-on:
5-(3-Methoxy-4-trifluormethoxy-phenyl)-3,6-dihydro-1,3,4-thiadiazin-2-on;
1-(4-Trifluormethoxyphenyl)-2-bromethan-1-on:
5-(4-Trifluormethoxy-phenyl)-3,6-dihydro-1,3,4-thiadiazin-2-on;
1-(3-Methoxy-4-difluormethoxy-phenyl)-2-bromethan-1-on:
5-(3-Methoxy-4-difluormethoxy-phenyl)-3,6-dihydro-1,3,4-thiadiazin-2-on;
1-[3-Methoxy-4-(1,1,2,2,-tetrafluorethoxy)-phenyl]-2-bromethan-1-on:
5-[3-Methoxy-4-(1,1,2,2-tetrafluorethoxy)-phenyl]-3,6-dihydro-1,3,4-thiadiazin-2-on;
1-(3-Methoxy-4-chlormethoxy-phenyl)-2-bromethan-1-on:
5-(3-Methoxy-4-chlormethoxy-phenyl)-3,6-dihydro-1,3,4-thiadiazin-2-on;
1-(3-Methoxy-4-trichlormethoxy-phenyl)-2-bromethan-1-on:
5-(3-Methoxy-4-trichlormethoxy-phenyl)-3,6-dihydro-1,3,4-thiadiazin-2-on;
1-(3-Methoxy-4-pentachlorethoxy-phenyl)-2-bromethan-1-on:
5-(3-Methoxy-4-pentachlorethoxy-phenyl)-3,6-dihydro-1,3,4-thiadiazin-2-on;
1-(4-Difluormethoxyphenyl)-2-bromethan-1-on:
5-(4-Difluormethoxyphenyl)-3,6-dihydro-1,3,4-thiadiazin-2-on.

### Beispiel 3

Eine Lösung von 5,4 g 5-(3-Methoxy-4-hydroxy-phenyl)-6-ethyl-3,6-dihydro-1,3,4-thiadiazin-2-on [erhältlich durch Umsetzung von 1-(4-Hydroxy-3-methoxyphenyl)-2-brombutan-1-on mit Hydrazinthioameisensäuremethylester] in THF wird nach Zugabe von einem Äquivalent 3-Iod-1,1,2,2,3-pentafluorpropan zwei Std. gekocht. Anschließend wird das Lösungsmittel im Vakuum entfernt und wie üblich aufgearbeitet. Man erhält 5-[3-Methoxy-4-(1,1,2,2,3-pentafluorpropoxy-phenyl]-6-ethyl-3,6-dihydro-1,3,4-thiadiazin-2-on.

Analog erhält man durch Veretherung der entsprechenden Mono- oder oder Dihydroxyphenyl-1,3,4-thiadiazinonderivate mit Polyfluoralkylhalogeniden:
5-(3-Methoxy-4-trifluormethoxy-phenyl)-3,6-dihydro-1,3,4-thiadiazin-2-on;
5-(4-Trifluormethoxy-phenyl)-3,6-dihydro-1,3,4-thiadiazin-2-on;
5-[Bis-3,4-(difluormethoxy)-phenyl]-3,6-dihydro-1,3,4-thiadiazin-2-on;
5-[3-Methoxy-4-(1,1,2-trifluorethoxy)-phenyl]-3,6-dihydro-1,3,4-thiadiazin-2-on;
5-[Bis-3,4-(dichlormethoxy)-phenyl]-3,6-dihydro-1,3,4-thiadiazin-2-on.

### Beispiel 4

Analog Beispiel 1 erhält man durch Umsetzung von Hydrazinthioameisensäuremethylester mit 1-[Bis-3,4-(difluormethoxy)-phenyl]-2-bromethan-1-on 5-[Bis-3,4-(difluormethoxy)-phenyl]-3,6-dihydro-1,3,4-thiadiazin-2-on.

Analog erhält man durch Umsetzung von Hydrazinthioameisensäuremethylester mit
1-[Bis-3,4-(trifluormethoxy)-phenyl]-2-bromethan-1-on:
5-[Di-(3,4-trifluormethoxy)-phenyl]-3,6-dihydro-1,3,4-thiadiazin-2-on;
1-[Bis-3,4-(1,2-difluorethoxy)-phenyl]-2-bromethan-1-on:
5-[Bis-3,4-(1,2-difluorethoxy)-phenyl]-3,6-dihydro-1,3,4-thiadiazin-2-on;
1-[3-Ethoxy-4-(1,1,2,2-tetrafluorethoxy)-phenyl]-2-bromethan-1-on:
5-[3-Ethoxy-4-(1,1,2,2-tetrafluorethoxy)-phenyl]-3,6-dihydro-1,3,4-thiadiazin-2-on;
1-[3-Methoxy-4-(1,2,2-Trichlorethoxy)-phenyl]-2-bromethan-1-on:
5-[3-Methoxy-4-(1,2,2-trichlorethoxy)-phenyl]-3,6-dihydro-1,3,4-thiadiazin-2-on.

### Beispiel 5

Analog Beispiel 1 erhält man durch Umsetzung von Hydrazinthioameisensäuremethylester mit:
1-(2-Methoxy-3-difluormethoxy-phenyl)-2-bromethan-1-on:
5-(2-Methoxy-3-difluormethoxy-phenyl)-3,6-dihydro-1,3,4-thiadiazin-2-on;
1-(2-Ethoxy-4-trifluormethoxy-phenyl)-2-bromethan-1-on:
5-(2-Ethoxy-4-trifluormethoxy-phenyl)-3,6-dihydro-1,3,4-thiadiazin-2-on;
1-(2-Trifluormethoxy-phenyl)-2-bromethan-1-on:
5-(2-Trifluormethoxy-phenyl)-3,6-dihydro-1,3,4-thiadiazin-2-on;
1-(2-Ethoxy-3-difluormethoxy-phenyl)-2-bromethan-1-on:
5-(2-Ethoxy-3-difluormethoxy-phenyl)-3,6-dihydro-1,3,4-thiadiazin-2-on;
1-[2-(1,1,2,2-Tetrafluorethoxy)-phenyl]-2-bromethan-1-on:
5-[2-(1,1,2,2-Tetrafluorethoxy)-phenyl]-3,6-dihydro-1,3,4-thiadiazin-2-on;
1-(3-Methoxy-5-chlormethoxy-phenyl)-2-bromethan-1-on:
5-(3-Methoxy-5-chlormethoxy-phenyl)-3,6-dihydro-1,3,4-thiadiazin-2-on;
1-(3-Methoxy-5-trichlormethoxy-phenyl)-2-bromethan-1-on:
5-(3-Methoxy-5-trichlormethoxy-phenyl)-3,6-dihydro-1,3,4-thiadiazin-2-on;
1-(2-Methoxy-4-pentachlorethoxy-phenyl)-2-bromethan-1-on:
5-(2-Methoxy-4-pentachlorethoxy-phenyl)-3,6-dihydro-1,3,4-thiadiazin-2-on;
1-(2-Difluormethoxy-phenyl)-2-bromethan-1-on:
5-(2-Difluormethoxy-phenyl)-3,6-dihydro-1,3,4-thiadiazin-2-on.

### Beispiel 6

Analog Beispiel 1 erhält man durch Umsetzung von Hydrazinthioameisensäuremethylester mit 1-[4-(2,2,2-Trifluorethoxy)-phenyl]-2-brombutan-1-on das 5-[4-(2,2,2-Trifluorethoxy)-phenyl]-6-ethyl-3,6-dihydro-1,3,4-thiadiazin-2-on, F. 102°.

Analog erhält man
mit 1-[3-Methoxy-4-(2,2,2-trifluorethoxy)-phenyl]-2-brombutan-1-on:
5-[3-Methoxy-4-(2,2,2-trifluorethoxy)-phenyl]-6-ethyl-3,6-dihydro-1,3,4-thiadiazin-2-on, F. 123-125°;
mit 1-[3-Methoxy-4-(2,2,2-trifluorethoxy)-phenyl]-2-bromethan-1-on:
5-[3-Methoxy-4-(2,2,2-trifluorethoxy)-phenyl]-3,6-dihydro-1,3,4-thiadiazin-2-on, F. 120°;
mit 1-[3-(2,2,2-Trifluorethoxy)-4-methoxy-phenyl]-2-brombutan-1-on:
5-[3-(2,2,2-Trifluorethoxy)-4-methoxy-phenyl]-6-ethyl-3,6-dihydro-1,3,4-thiadiazin-2-on, F. 120-121°;
mit 1-[3-Difluormethoxy-4-methoxy-phenyl)-2-brombutan-1-on:
5-(3-Difluormethoxy-4-methoxy-phenyl)-6-ethyl-3,6-dihydro-1,3,4-thiadiazin-2-on, F. 105°.

### Beispiel 7

Durch chromatographische Enantiomerentrennung unter Verwendung einer chiralen stationären Phase (ChiraSpher®) erhält man ausgehend von racemischem 5-(3-Methoxy-4-difluormethoxy-phenyl)-6-ethyl-3,6-dihydro-1,3,4-thiadiazin-2-on [F. 97°; erhältlich nach Bsp. 1]:
(+)-5-(3-Methoxy-4-difluormethoxy-phenyl)-6-ethyl-3,6-dihydro-1,3,4-thiadiazin-2-on
und
(-)-5-(3-Methoxy-4-difluormethoxy-phenyl)-6-ethyl-3,6-dihydro-1,3,4-thiadiazin-2-on.

Analog erhält man ausgehend von racemischem 5-[3-(2,2,2-Trifluorethoxy)-4-methoxy-phenyl]-6-ethyl-3,6-dihydro-1,3,4-thiadiazin-2-on [F. 120-121°; erhältlich nach Bsp. 6]:
(+)-5-[3-(2,2,2-Trifluorethoxy)-4-methoxy-phenyl]-6-ethyl-3,6-dihydro-1,3,4-thiadiazin-2-on
und
(-)-5-[3-(2,2,2-Trifluorethoxy)-4-methoxy-phenyl]-6-ethyl-3,6-dihydro-1,3,4-thiadiazin-2-on.

Die nachstehenden Beispiele betreffen pharmazeutische Zubereitungen, die Verbindungen der Formel I oder ihre Säureadditionssalze enthalten:

### Beispiel A: Tabletten

Ein Gemisch von 1 kg 5-(3-Methoxy-4-difluormethoxy-phenyl)-6-ethyl-3,6-dihydro-1,3,4-thiadiazin-2-on, 10 kg Lactose, 6 kg mikrokristalline Cellulose, 6 kg Kartoffelstärke, 1 kg Polyvinylpyrrolidon, 0,8 kg Talk und 0,1 kg Magnesiumstearat wird in üblicher Weise zu Tabletten gepreßt, derart, daß jede Tablette 10 mg Wirkstoff enthält.

### Beispiel B: Dragees

Analog Beispiel A werden Tabletten gepreßt, die anschließend in üblicher Weise mit einem Überzug aus Saccharose, Kartoffelstärke, Talk, Tragant und Farbstoff überzogen werden.

### Beispiel C: Kapseln

1 kg 5-(3-Methoxy-4-difluormethoxy-phenyl)-3,6-dihydro-1,3,4-thiadiazin-2-on wird in üblicher Weise in Hartgelatinekapseln gefüllt, so daß jede Kapsel 5 mg Wirkstoff enthält.

### Beispiel D: Ampullen

Eine Lösung von 1 kg 5-(3-Methoxy-4-difluormethoxy-phenyl)-3,6-dihydro-1,3,4-thiadiazin-2-on in 30 l 1,2-Propandiol wird steril filtriert, in Ampullen abgefüllt, unter sterilen Bedingungen lyophilisiert und steril verschlossen. Jede Ampulle enthält 2 mg Wirkstoff.

Analog sind Tabletten, Dragees, Kapseln und Ampullen erhältlich, die einen oder mehrere der übrigen Wirkstoffe der Formel I und/oder ihre physiologisch unbedenklichen Säureadditionssalze enthalten.

## Patentansprüche (Patentansprüche für folgende(n) Vertragsstaat(en): AT, BE, CH, LI, DE, DK, FR, GB, GR, IE, IT, LU, MC, NL, PT, SE)

1. Thiadiazinonderivate der Formel I 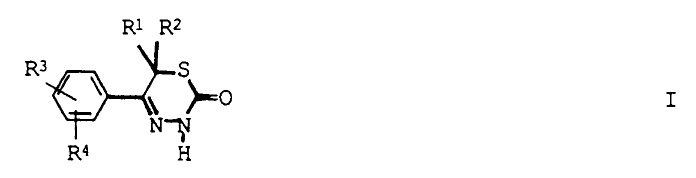 worin
R¹ und R² jeweils unabhängig voneinander H oder A,
R³ H, OA oder O-CₘH₂ₘ₊₁₋ₙXₙ,
R⁴ -O-CₘH₂ₘ₊₁₋ₙXₙ,
X F oder Cl,
A Alkyl mit 1-6 C-Atomen,
m 1, 2, 3, 4, 5 oder 6 und
n 1, 2, 3, 4, 5, 6, 7, 8, 9, 10, 11, 12 oder 13
bedeuten,
sowie deren Salze.

2. a) 5-(3-Methoxy-4-difluormethoxy-phenyl)-6-ethyl-3,6-dihydro-1,3,4-thiadiazin-2-on;
b) 5-(3-Methoxy-4-difluormethoxyphenyl)-3,6-dihydro-1,3,4-thiadiazin-2-on.

3. Verfahren zur Herstellung von Thiadiazinonen der Formel I nach Anspruch 1 sowie von deren Salzen, dadurch gekennzeichnet, daß man eine Verbindung der Formel II 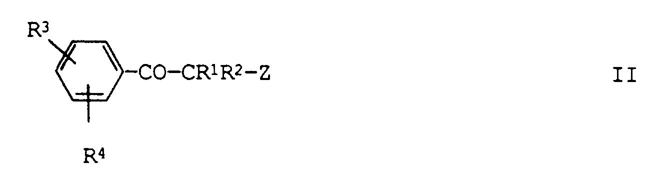 worin R¹, R², R³ und R⁴ die angegebenen Bedeutungen haben und
Z Br, Cl, I oder eine reaktionsfähig veresterte OH-Gruppe bedeutet,
mit einer Verbindung der Formel III
H₂N-NH-CS-OR⁵ III
worin
R⁵ A, Ammonium, Na oder K bedeutet
und A die angegebene Bedeutung hat,
umsetzt,
und/oder daß man gegebenenfalls eine Verbindung, die der Formel I entspricht, jedoch anstelle von R³ und/oder R⁴ eine oder zwei freie OH-Gruppen enthält, mit einer Verbindung der Formel R³-Z bzw. R⁴-Z, worin R³, R⁴ und Z die angegebenen Bedeutungen haben, umsetzt und/oder eine Base der Formel I durch Behandeln mit einer Säure in eines ihrer Salze umwandelt.

4. Verfahren zur Herstellung pharmazeutischer Zubereitungen, dadurch gekennzeichnet, daß man eine Verbindung der Formel I nach Anspruch 1 und/oder eines ihrer physiologisch unbedenklichen Salze zusammen mit mindestens einem festen, flüssigen oder halbflüssigen Träger- oder Hilfsstoff in eine geeignete Dosierungsform bringt.

5. Pharmazeutische Zubereitung, gekennzeichnet durch einen Gehalt an mindestens einer Verbindung der Formel 1 nach Anspruch 1 und/oder einem ihrer physiologisch unbedenklichen Salze.

6. Verwendung einer Verbindung der Formel I nach Anspruch 1 und/oder eines ihrer physiologisch unbedenklichen Salze zur Herstellung eines Arzneimittels zur Bekämpfung von Krankheiten.

## Patentansprüche (Patentansprüche für folgende(n) Vertragsstaat(en): ES)

1. Verfahren zur Herstellung von Thiadiazinonderivaten der Formel I 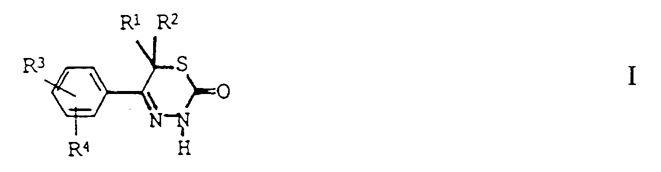 worin
R¹ und R² jeweils unabhängig voneinander H oder A,
R³ H, OA oder O-CₘH₂ₘ₊₁₋ₙXₙ,
R⁴ -O-CₘH₂ₘ₊₁₋ₙXₙ,
X F oder Cl,
A Alkyl mit 1-6 C-Atomen,
m 1, 2, 3, 4, 5 oder 6 und
n 1, 2, 3, 4, 5, 6, 7, 8, 9, 10, 11, 12 oder 13
bedeuten,
sowie von deren Salzen, dadurch gekennzeichnet, daß man eine Verbindung der Formel II 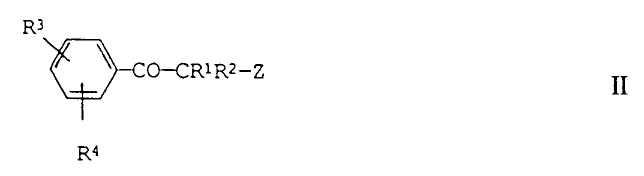 worin
R¹, R², R³ und R⁴ die angegebenen Bedeutungen haben und
Z Br, Cl, I oder eine reaktionsfähig veresterte OH-Gruppe bedeutet,
mit einer Verbindung der Formel III
H₂N-NH-CS-OR⁵ III
worin
R⁵ A, Ammonium, Na oder K bedeutet,
und A die angegebene Bedeutung hat,
umsetzt,
und/oder daß man gegebenenfalls eine Verbindung, die der Formel I entspricht, jedoch anstelle von R³ und/oder R⁴ eine oder zwei freie OH-Gruppen enthält, mit einer Verbindung der Formel R³-Z bzw. R⁴-Z, worin R³, R⁴ und Z die angegebenen Bedeutungen haben, umsetzt und/oder eine Base der Formel I durch Behandeln mit einer Säure in eines ihrer Salze umwandelt.

2. Verfahren zur Herstellung von
a) 5-(3-Methoxy-4-difluormethoxy-phenyl)-6-ethyl-3,6-dihydro-1,3,4-thiadiazin-2-on;
b) 5-(3-Methoxy-4-difluormethoxy-phenyl)-3,6-dihydro-1,3,4-thiadiazin-2-on
gemäß Anspruch 1.

3. Verfahren zur Herstellung pharmazeutischer Zubereitungen, dadurch gekennzeichnet, daß man eine Verbindung der Formel I nach Anspruch 1 und/oder eines ihrer physiologisch unbedenklichen Salze zusammen mit mindestens einem festen, flüssigen oder halbflüssigen Träger- oder Hilfsstoff in eine geeignete Dosierungsform bringt.

## Claims (Claims for the following Contracting State(s): AT, BE, CH, LI, DE, DK, FR, GB, GR, IE, IT, LU, MC, NL, PT, SE)

1. Thiadiazinone derivative of the fomula I 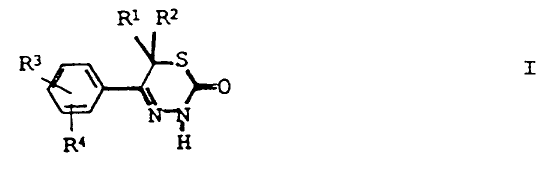 in which
R¹ and R² are each independently of one another H or A,
R³ is H, OA or O-CₘH₂ₘ₊₁₋ₙXₙ,
R⁴ is -O-CₘH₂ₘ₊₁₋ₙXₙ,
X is F or Cl,
A is alkyl having 1-6 C atoms,
m is 1, 2, 3, 4, 5 or 6 and
n is 1, 2, 3, 4, 5, 6, 7, 8, 9, 10, 11, 12 or 13,
and their salts.

2. a) 5- (3-Methoxy-4-difluoromethoxyphenyl)-6-ethyl-3,6-dihydro-1,3,4-thiadiazin-2-one;
b) 5-(3-methoxy-4-difluoromethoxyphenyl)-3,6-dihydro-1,3,4-thiadiazin-2-one.

3. Process for the preparation of thiadiazinones of the formula I according to Claim 1 and of their salts, characterised in that a compound of the formula II 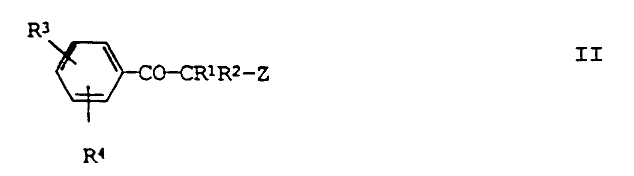 in which R¹, R², R³ and R⁴ have the meanings indicated and Z is Br, Cl, I or a reactive esterified OH group, is reacted with a compound of the formula III
H₂H-NH-CS-OR⁵ III
in which
R⁵ is A, ammonium, Na or K
and A has the meaning indicated,
and/or in that a compound which corresponds to the formula I, but instead of R³ and/or R⁴ contains one or two free OH groups, is optionally reacted with a compound of the formula R³-Z or R⁴-Z, in which R³, R⁴ and Z have the meanings indicated, and/or a base of the formula I is converted into one of its salts by treatment with an acid.

4. Process for the production of pharmaceutical preparations, characterised in that a compound of the formula I according to Claim 1 and/or one of its physiologically acceptable salts is brought into a suitable dosage form together with at least one solid, liquid or semi-liquid excipient or auxiliary.

5. Pharmaceutical preparation, characterised in that it contains at least one compound of the formula I according to Claim 1 and/or one of its physiologically acceptable salts.

6. Use of a compound of the formula I according to Claim 1 and/or of one of its physiologically acceptable salts for the production of a medicament for the control of diseases.

## Claims (Claims for the following Contracting State(s): ES)

1. Process for the preparation of thiadiazinones of the formula I 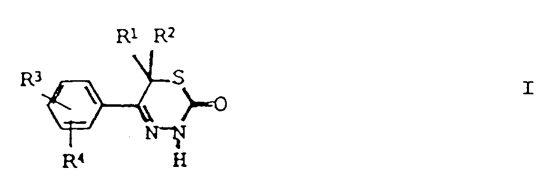 in which
R¹ and R² are each independently of one another H or A,
R³ is H, OA or O-CₘH₂ₘ₊₁₋ₙXₙ,
R⁴ is -O-CₘH₂ₘ₊₁₋ₙXₙ,
X is F or Cl,
A is alkyl having 1-6 C atoms,
m is 1, 2, 3, 4, 5 or 6 and
n is 1, 2, 3, 4, 5, 6, 7, 8, 9, 10, 11, 12 or 13,
and their salts
characterised in that a compound of the formula II 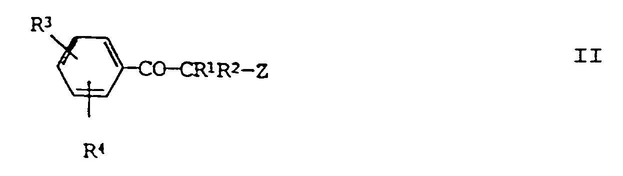 in which R¹, R², R³ and R⁴ have the meanings indicated and
Z is Br, Cl, I or a reactive esterified OH group, is reacted with a compound of the formula III
H₂H-NH-CS-OR⁵ III
in which
R⁵ is A, ammonium, Na or K
and A has the meaning indicated,
and/or in that a compound which corresponds to the formula I, but instead of R³ and/or R⁴ contains one or two free OH groups, is optionally reacted with a compound of the formula R³-Z or R⁴-Z, in which R³, R⁴ and Z have the meanings indicated, and/or a base of the formula I is converted into one of its salts by treatment with an acid.

2. Process for the preparation of
a) 5-(3-Methoxy-4-difluoromethoxyphenyl)-6-ethyl-3,6-dihydro-1,3,4-thiadiazin-2-one;
b) 5-(3-methoxy-4-difluoromethoxyphenyl)-3,6-dihydro-1,3,4-thiadiazin-2-one according to Claim 1.

3. Process for the production of pharmaceutical preparations, characterised in that a compound of the formula I according to Claim 1 and/or one of its physiologically acceptable salts is brought into a suitable dosage form together with at least one solid, liquid or semi-liquid excipient or auxiliary.

## Revendications (Revendications pour l'(les) Etat(s) contractant(s) suivant(s): AT, BE, CH, LI, DE, DK, FR, GB, GR, IE, IT, LU, MC, NL, PT, SE)

1. Dérivés de thiadiazinones de formule I 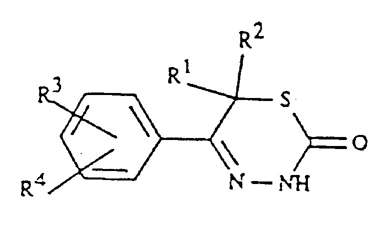 où
R¹ et R² représentent, indépendamment l'un de l'autre, H ou A,
R³ H, OA ou O-CₘH₂ₘ₊₁₋ₙXₙ,
R⁴ -O-CₘH₂ₘ₊₁₋ₙXₙ,
X F ou Cl,
A un alkyle ayant 1 à 6 atomes de C,
m est égal à 1, 2, 3, 4, 5 ou 6 et
n est égal à 1, 2, 3, 4, 5, 6, 7, 8, 9, 10, 11, 12 ou 13,
ainsi que leurs sels.

2. a) La 5-(3-méthoxy-4-difluorométhoxyphényl)-6-éthyl-3,6-dihydro-1,3,4-thiadiazin-2-one ;
b) La 5-(3-méthoxy-4-difluorométhoxyphényl)-3,6-dihydro-1,3,4-thiadiazin-2-one .

3. Procédé pour la préparation des thiadiazinones de formule 1 selon la revendication 1, ainsi que de leurs sels, caractérisé en ce que l'on fait réagir un composé de formule II 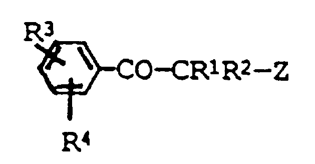 où R¹, R², R³ et R⁴ ont les significations qui ont été indiquées et
Z représente Br, Cl, I ou un groupe 0H estérifié réactif,
avec un composé de formule III
H₂N-NH-CS-0R⁵ III
où
R⁵ représente A, l'ammonium, Na ou K,
et A a la signification qui a été indiquée,
et/ou en ce que l'on fait éventuellement réagir un composé correspondant à la formule I, mais qui à la place de R³ et/ou de R⁴ contient un ou deux groupes 0H libres, avec un composé de formule R³-Z ou R⁴-Z où R³, R⁴ et Z ont les significations qui ont été indiquées et/ou en ce que l'on transforme une base de formule I, par traitement avec un acide, en l'un de ses sels.

4. Procédé pour la préparation de compositions pharmaceutiques, caractérisé en ce que l'on met sous une forme de dosage appropriée un composé de formule I selon la revendication 1 et/ou l'un de ses sels physiologiquement acceptables, associé à au moins un support ou adjuvant , solide, liquide ou semi-liquide .

5. Composition pharmaceutique, caractérisée en ce qu'elle contient au moins un composé de formule I selon la revendication 1 et/ou l'un de ses sels physiologiquement acceptables.

6. Utilisation d'un composé de formule I selon la revendication 1 et/ou de l'un de ses sels physiologiquement acceptables pour la préparation d'un médicament pour lutter contre les maladies.

## Revendications (Revendications pour l'(les) Etat(s) contractant(s) suivant(s): ES)

1. Procédé pour la préparation des thiadiazinones de formule I 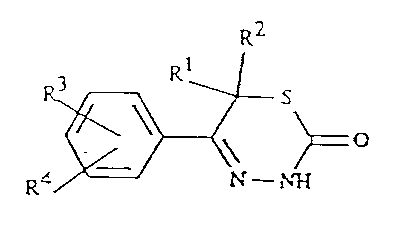 où
R¹ et R² répresentent, indépendamment l'un de l'autre, H ou A,
R³ H , OA ou O-CₘH₂ₘ₊₁₋ₙXₙ,
R⁴ -O-CₘH₂ₘ₊₁₋ₙXₙ,
X F ou Cl,
A un alkyle ayant 1 à 6 atomes de C,
m est égal à 1, 2, 3, 4, 5 ou 6 et
n est égal à 1, 2, 3, 4, 5, 6, 7, 8, 9, 10, 11, 12 ou 13,
ainsi que leurs sels caractérisé en ce que l'on fait réagir un
composé de formule II 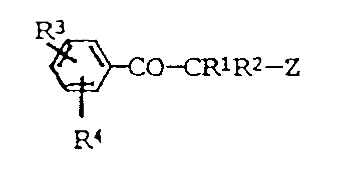 où R¹, R², R³ et R⁴ ont les significations qui ont été indiquées et
Z représente Br, Cl, I ou un groupe OH estérifié réactif,
avec un composé de formule III
H₂N-NH-CS-0R⁵ III
où
R⁵ représente A, l'ammonium, Na ou K,
et A a la signification qui a été indiquée,
et/ou en ce que l'on fait éventuellement réagir un composé correspondant à la formule I, mais qui à la place de R3 et/ou de R⁴ contient un ou deux groupes 0H libres, avec un composé de formule R³-Z ou R⁴-Z où R³, R⁴ et Z ont les significations qui ont été indiquées et/ou en ce que l'on transforme une base de formule I, par traitement avec un acide, en l'un de ses sels.

2. Procédé pour la préparation
a) La 5-(3-méthoxy-4-difluorométhoxyphényl)-6-éthyl-3,6-dihydro-1,3,4-thiadiazin-2-one ;
b) La 5-(3-méthoxy-4-difluorométhoxyphényl)-3,6-dihydro-1,3,4-thiadiazin-2-one
selon la revendication 1

3. Procédé pour la préparation de compositions pharmaceutiques, caractérisé en ce que l'on met sous me forme de dosage appropriée un composé de formule I selon la revendication 1 et/ou l'un de ses sels physiologiquement acceptables, associé à au moins un support ou adjuvant , solide, liquide ou semi-liquide.
